# EUROPÄISCHE PATENTSCHRIFT

(11) **EP 1 490 056 B1**
(45) Veröffentlichungstag und Bekanntmachung des Hinweises auf die Patenterteilung: **30.08.2006**
(21) Anmeldenummer: 03709758.1
(22) Anmeldetag: 07.03.2003
(51) Int. Cl.: A61K 31/402, A61K 31/451, C07D 207/22, C07D 211/72, C07D 401/12, C07D 453/06

(54) **N-'4-(2-IMINO-PYRROLIDIN-1-YL)PHENYL)-ACETEMID-UND ENTSPRECHENDE PIPERIDINDERIVATE ALS FAKTOR XA INHIBITOREN ZUR BEHANDLUNG VON THROMBOEMBOLISCHEN ERKRANKUNGEN**
N-'4-(2-IMINO-PYRROLIDIN-1-YL)PHENYL)-ACETAMIDE AND CORRESPONDING PIPERIDINE DERIVATIVES AS FACTOR XA INHIBITORS FOR THE TREATMENT OF THROMBO-EMBOLIC DISEASES
N-'4-(2-IMINO-PYRROLIDIN-1-YL)PHENYL)-ACETEMIDE ET DERIVES DE PIPERIDINE CORRESPONDANT SERVANT D'INHIBITEURS DE FACTEUR XA POUR TRAITER DES MALADIES THROMBOEMBOLIQUES

(30) Priorität: 04.04.2002 DE 10214832
(43) Veröffentlichungstag der Anmeldung: 29.12.2004
(73) Patentinhaber: Merck Patent GmbH, 64293 Darmstadt (DE)
(72) Erfinder: CEZANNE, Bertram, 64546 Mörfelden-Walldorf (DE); DORSCH, Dieter, 64372 Ober-Ramstadt (DE); MEDERSKI, Werner, 64673 Zwingenberg (DE); TSAKLAKIDIS, Christos, 69469 Weinheim (DE); BARNES, Christopher, 65812 Bad Soden (DE); GLEITZ, Johannes, 64293 Darmstadt (DE)
(86) Internationale Anmeldenummer: PCT/EP2003/002349
(87) Internationale Veröffentlichungsnummer: WO 2003/084533

(56) Entgegenhaltungen:
- WO-A-00/71493
- WO-A-00/71509
- DE-A- 10 027 025
- DE-A- 10 102 322
- US-A- 4 556 674

## Beschreibung

Die Erfindung betrifft Verbindungen der Formel I 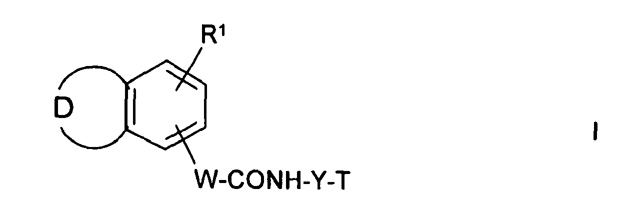
- D: fehlt oder
-CH=N-CH=CH-, -NH-N=CH-, -O-N=CH- oder
-CH₂-CH₂-CH₂-CH₂-,
und wobei, falls D vorhanden ist, zusätzlich an D eine einfache Substitution durch NH₂ auftreten kann,
- R¹: H oder CH₂NH₂,
- W: -OC(R^{2a})₂- oder -NR²C(R^{2a})₂-,
- R^{2a}: H, A' oder Ar',
- A': Alkyl mit 1, 2, 3, 4, 5 oder 6 C-Atomen und wobei 1-7 H-Atome durch F ersetzt sein können,
- Ar': unsubstituiertes oder ein- oder zweifach durch Hal substituiertes Phenyl oder Benzyl,
- Y: unsubstituiertes oder ein- oder zweifach durch A, Cl oder F substituiertes Phenylen,
- T: einfach durch =NR^{2b}, =S oder =NOR^{2b} substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H*-Pyridazin-2-yl, Azepan-1-yl, 2-Aza-bicyclo[2.2.2]-octan-2-yl,
- R^{2b}: H, -CH₂CH₂NA'₂, OH oder OA",
- A": Methyl, Ethyl, Propyl, Isopropyl oder Butyl
bedeuten,
sowie ihre pharmazeutisch verwendbaren Salze, Solvate und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I können weiterhin Inhibitoren der Gerinnungsfaktoren Faktor VIIa, Faktor IXa und Thrombin der Blutgerinnungskaskade sein.

Aromatische Amidinderivate mit antithrombotischer Wirkung sind z.B. aus der EP 0 540 051 B1, WO 98/28269, WO 00/71508, WO 00/71511, WO 00/71493, WO 00/71507, WO 00/71509, WO 00/71512, WO 00/71515 oder WO 00/71516 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)-phenylalkyl]-azaheterocyclylamide als Faktor Xa Inhibitoren sind in WO 96/40679 beschrieben.

Andere gerinnungshemmende Faktor Xa Inhibitoren kennt man aus DE 100 27 025 A1 und DE 101 02 322 A1. In US 4,556,674 sind Pyrrolidinderivate zur Behandlung cardiovaskulärer Probleme beschrieben.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor Vlla, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in *Circulation* **1996, *94,*** 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in *Thrombosis and Haemostasis* **1990,** 63, 220-223 beschrieben.

Die Messung der Inhibierung von Faktor Xa kann z.B. nach der Methode von T. Hara et al. in *Thromb. Haemostas.* **1994,** 79, 314-319 erfolgen.

Der Gerinnungsfaktor VIIa initiiert nach Bindung an Tissue Faktor den extrinsischen Teil der Gerinnungskaskade und trägt zur Aktivierung des Faktors X zu Faktor Xa bei. Eine Inhibierung von Faktor VIIa verhindert somit die Entstehung des Faktors Xa und damit eine nachfolgende Thrombinbildung.
Die Inhibierung des Faktors VIIa durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIa wird z.B. von H. F. Ronning et al, in *Thrombosis Research* **1996,** 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird. Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in *Journal of Biological Chemistry* **1998,** 273, 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt.

Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo,* oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F.Morales-Asin et al., Headache, 40, 2000, 45-47).

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel I nach den Ansprüchen 1-20 sowie ihrer pharmazeutisch verwendbaren Salze, Solvate und Stereoisomere, dadurch gekennzeichnet, daß man
a) zur Herstellung einer Verbindung der Formel I, worin W -OC(R²)₂- oder -NR²C(R²)₂- bedeutet,
   eine Verbindung der Formel II 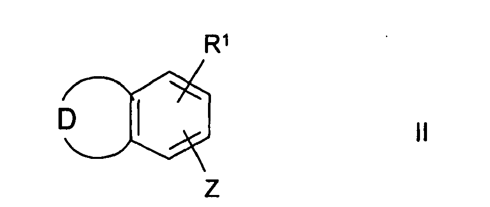 worin
   Z OH oder NHR² bedeutet
   und R¹, R² und D die in Anspruch 1 angegebenen Bedeutungen haben, mit der Maßgabe, daß falls eine weitere OH- und/oder Aminogruppe vorliegt, diese geschützt ist,
   mit einer Verbindung der Formel III

   L-C(R²)₂-CONH-Y-T III

   worin
   L Cl, Br oder I bedeutet und R², Y und T die in Anspruch 1 angegebenen Bedeutungen haben,
   umsetzt,
   und anschließend gegebenenfalls eine Schutzgruppe abspaltet,
b) zur Herstellung einer Verbindung der Formel I,
   eine Verbindung der Formel IV 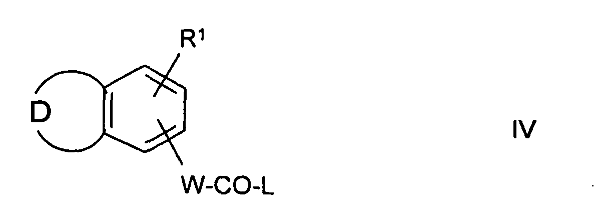 worin
   L Cl, Br, I oder eine freie oder reaktionsfähig funktionell abgewandelte OH-Gruppe bedeutet bedeutet
   und R¹, D und W die in Anspruch 1 angegebenen Bedeutungen haben, mit der Maßgabe, daß falls eine weitere OH- und/oder Aminogruppe vorliegt, diese geschützt ist,
   mit einer Verbindung der Formel V

   Z'-Y-T V

   worin
   Z' NHR² oder NHR²C(R³)₂ bedeutet
   und R², Y und T die in Anspruch 1 angegebenen Bedeutungen haben, umsetzt,
   und anschließend gegebenenfalls eine Schutzgruppe abspaltet,
c) und/oder daß man in einer Verbindung der Formel I einen Rest T und/oder R¹ in einen anderen Rest T und/oder R¹ umwandelt,
   indem man beispielsweise
   i) eine Sulfanylverbindung in eine Iminoverbindung umwandelt,
   ii) eine Aminoschutzgruppe abspaltet,
      und/oder
      eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, wie z.B. A, gilt, daß deren Bedeutungen unabhängig voneinander sind.
Vor- und nachstehend haben die Reste bzw. Parameter D, W, Y, T, R¹ die bei der Formel I angegebenen Bedeutungen, falls nicht ausdrücklich etwas anderes angegeben ist.

D bedeutet besonders bevorzugt -CH=N-CH=CH-, -NH-N=CH-, -O-N=CH- oder -CH₂-CH₂-CH₂-CH₂-, wobei zusätzlich an D eine einfache Substitution durch NH₂ auftreten kann, oder D fehlt.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Hal bedeutet vorzugsweise F, Cl oder Br, aber auch I.

R¹ bedeutet vorzugsweise H oder CH₂NH₂.

W bedeutet besonders bevorzugt -OCHR^{2a}- oder -NHCHR^{2a}-,
worin
- R^{2a}: A' oder Ar',
- A': Alkyl mit 1, 2, 3, 4, 5 oder 6 C-Atomen und wobei 1-7 H-Atome durch F ersetzt sein können und
- Ar': unsubstituiertes oder ein- oder zweifach durch Hal substituiertes Phenyl oder Benzyl,
bedeuten.

Y bedeutet besonders bevorzugt unsubstituiertes oder einfach durch A, Cl oder F substituiertes 1,4-Phenylen.
Y bedeutet insbesondere unsubstituiertes oder einfach durch Methyl, Ethyl, Propyl, Cl oder F substituiertes 1,3- oder 1,4-Phenylen.

T bedeutet insbesondere ein- oder zweifach durch =NR², =S oder =NOR² substituiertes Piperidin-1-yl, Pyrrolidin-1-yl, 1 H-Pyridin-1-yl, Morpholin-4-yl, Piperazin-1-yl, 1,3-Oxazolidin-3-yl, 2*H*-Pyridazin-2-yl, Azepan-1-yl, 2-Azabicyclo[2.2.2]-octan-2-yl.

T bedeutet weiterhin besonders bevorzugt z.B. 2-Imino-piperidin-1-yl, 2-Imino-pyrrolidin-1-yl, 2-Imino-1*H*-pyridin-1-yl, 3-Imino-morpholin-4-yl, 4-Imino-1*H*-pyridin-1-yl, 2,6-Diimino-piperidinl-yl, 2-Imino-piperazin-1-yl, 2,6-Diimino-piperazin-1-yl, 2,5-Diimino-pyrrolidin-1-yl, 2-Imino-1,3-oxazolidin-3-yl, 3-Imino-2*H*-pyridazin-2-yl, 2-Imino-azepan-1-yl, 2-Hydroxy-6-iminopiperazin-1-yl oder 2-Methoxy-6-imino-piperazin-1-yl, ganz besonders bevorzugt ist 2-Imino-piperidin-1-yl, sowie die entsprechenden Hydroxyimino-, Thioxo- und =N-(CH₂)₁₋₃NA'₂ - Derivate, wobei A' Alkyl mit 1, 2, 3, 4, 5, oder 6 C-Atomen bedeutet.

Die Verbindungen der Formel I können ein oder mehrere chirale Zentren besitzen und daher in verschiedenen stereoisomeren Formen vorkommen. Die Formel I umschließt alle diese Formen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II, III, IV und V sind in der Regel bekannt. Sind sie neu, so können sie aber nach an sich bekannten Methoden hergestellt werden.

Nach folgendem Schema lassen sich alle Verbindungen der folgenden Formel VI (mit R = H oder Methyl; n = 3, 4 oder 5) synthetisieren. 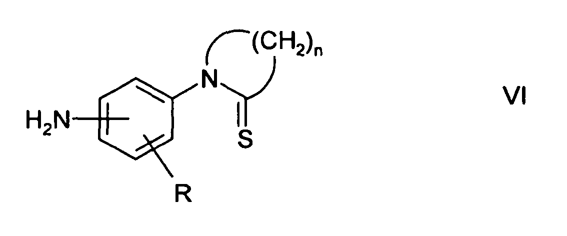 Z.B. Synthese von 1-(4-Amino-2-methylphenyl)-piperidin-2-thion: 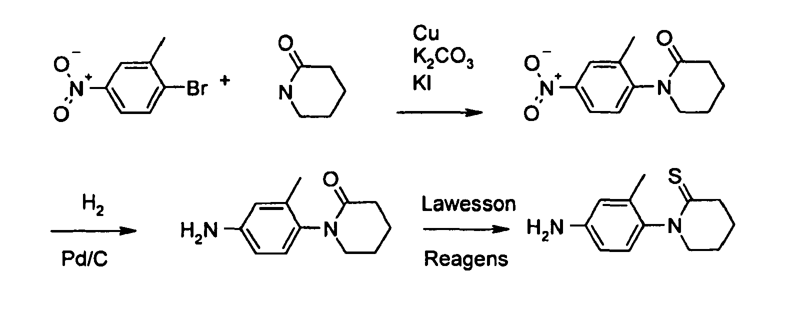 Alternativsynthese: 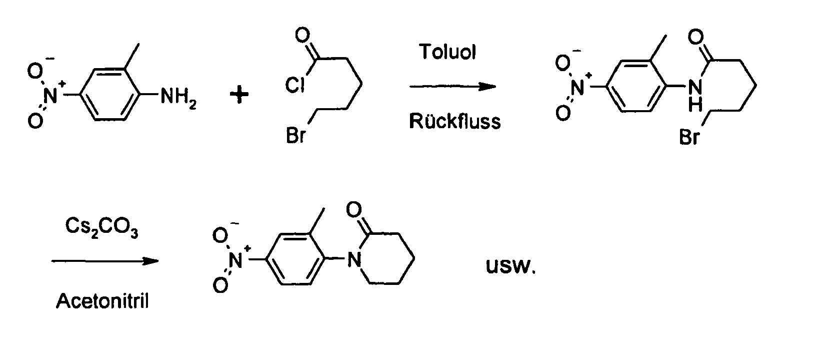 Synthese des Phenylpiperidin-thion-bausteins ohne Methylgruppe:

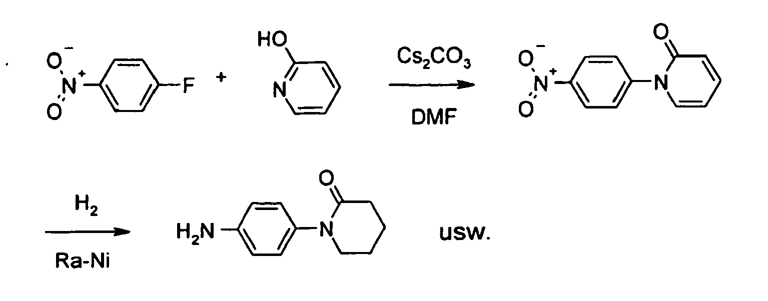

Gegenstand der Erfindung sind daher auch Verbindungen der Formel VI sowie deren Salze.

Eine Base der Formel VI kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon-oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure.

Verbindungen der Formel I können vorzugsweise erhalten werden, indem man Verbindungen der Formel II mit Verbindungen der Formel III umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Phenolkomponente der Formel II bzw. des Alkylierungsderivates der Formel III kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykol-monomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können weiter vorzugsweise erhalten werden, indem man Verbindungen der Formel IV mit Verbindungen der Formel V umsetzt.
Die Umsetzung erfolgt in der Regel in einem inerten Lösungsmittel und unter Bedingungen wie oben angegeben.

In den Verbindungen der Formel IV bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel IV.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich die oben genannten.

Verbindungen der Formel I können ferner erhalten werden, indem man Verbindungen der Formel I au einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOR" tragen, worin R" eine Hydroxyschutzgruppe bedeutet.
Bevorzugte Ausgangsstoffe sind auch die Oxadiazolderivate, die in die entsprechenden Amidinoverbindungen überführt werden können.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl-oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC (tert.-Butyloxycarbonyl), 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, 4-Methoxybenzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ, Benzyl oder die Freisetzung der Amidinogruppe aus ihrem Oxadiazolderivat)) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Trifluormethylbenzol, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ester können z.B. mit Essigsäure oder mit NaOH oder KOH in Wasser, Wasser-THF oder Wasser-Dioxan bei Temperaturen zwischen 0 und 100° verseift werden.

Ferner kann man freie Aminogruppen in üblicher Weise mit einem Säurechlorid oder -anhydrid acylieren oder mit einem unsubstituierten oder substituierten Alkylhalogenid alkylieren, oder mit CH₃-C(=NH)-OEt umsetzen, zweckmäßig in einem inerten Lösungsmittel wie Dichlormethan oder THF und /oder in Gegenwart einer Base wie Triethylamin oder Pyridin bei Temperaturen zwischen -60 und +30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium-oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Derivate, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Gegenstand der Erfindung ist ferner die Verwendung von Verbindungen der Formel I und/oder ihrer pharmazeutisch verwendbaren Derivate, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit
Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel:
Ethylacetat/Methanol 9:1.
- Massenspektrometrie (MS):: EI (Elektronenstoß-Ionisation) M⁺
FAB (Fast Atom Bombardment) (M+H)⁺
ESI (Electrospray lonization) (M+H)⁺ (wenn
nichts anderes angegeben)

### Beispiel 1

### Herstellung eines Aminbausteines:

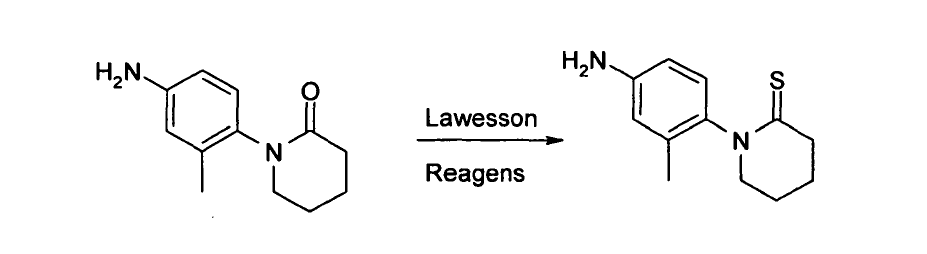

10 g (48.95 mmol) 1-(4-Amino-2-methyl-phenyl)-piperidin-2-on werden zusammen mit 9.9 g (24.48 mmol) 2,4-Bis-(4-methoxy-phenyl)-[1,3,2,4]dithiadiphosphetan 2,4-disulfide (Lawesson - Reagens) in 70ml wasserfreiem Toluol zum Sieden erhitzt. Nach 40 min. wird das Lösungsmittel entfernt und der Rückstand in Dichlormethan (DCM)/ 1M wässriger Salzsäure aufgenommen. Nach mehrmaligem Waschen mit DCM stellt man mit konz. Natronlauge auf einen pH-Wert von 12 ein. Extraktion mit DCM, Trocknen über Na₂SO₄ und Eindampfen des Lösungsmittels liefern 9.25g (41.98 mmol) 1-(4-Amino-2-methyl-phenyl)-piperidin-2-thion.

### Herstellung eines Säurebausteines:

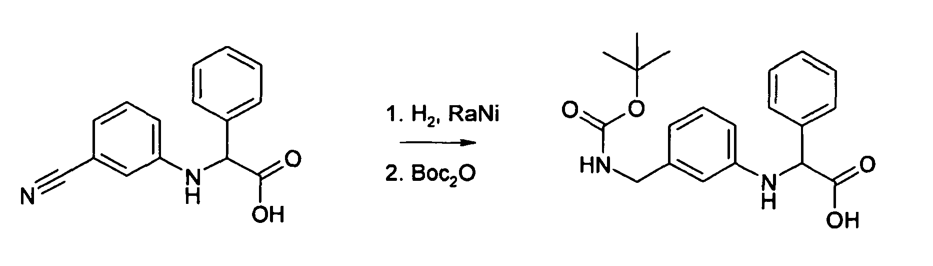

5 g (19.82 mmol) (R,S)-(3-Cyan-phenylamino)-phenylessisäure werden in 50 ml ammoniakalischem Methanol unter Druck an Raney-Nickel bei 50°C bis zum vollständigem Umsatz hydriert. Nach Filtration wird das Lösungsmittel entfernt. Das Rohprodukt wird in 80ml 1,4-Dioxan / Wasser (1:1) gelöst und mit 3.4 g (32.08 mmol) Na₂CO₃ versetzt. Anschließend tropft man unter Kühlung im Eisbad eine Lösung von 3.5g (16.04 mmol) Di-tert-butyl-dicarbonat in 40ml 1,4-Dioxan zum Reaktionsgemisch. Nach 19 Stunden wird das Dioxan abdestilliert und die wässrige Phase mit 2 M wässriger Salzsäurelösung auf pH =3.5 eingestellt. Nach Extraktion mit Ethylacetat, Trocknen über Na₂SO₄ und Eindampfen der Extrakte erhält man 4.51 g (10.78 mmol) (R,S)-[3-(*tert*-Butoxycarbonylamino-methyl)-phenylamino]-phenylessigsäure.

### Herstellung von 2-(3-Aminomethyl-phenylamino)-N-[3-chlor-4-(2-imino-pyrrolidin-1-yl)-phenyl]-2-phenyl acetamid :

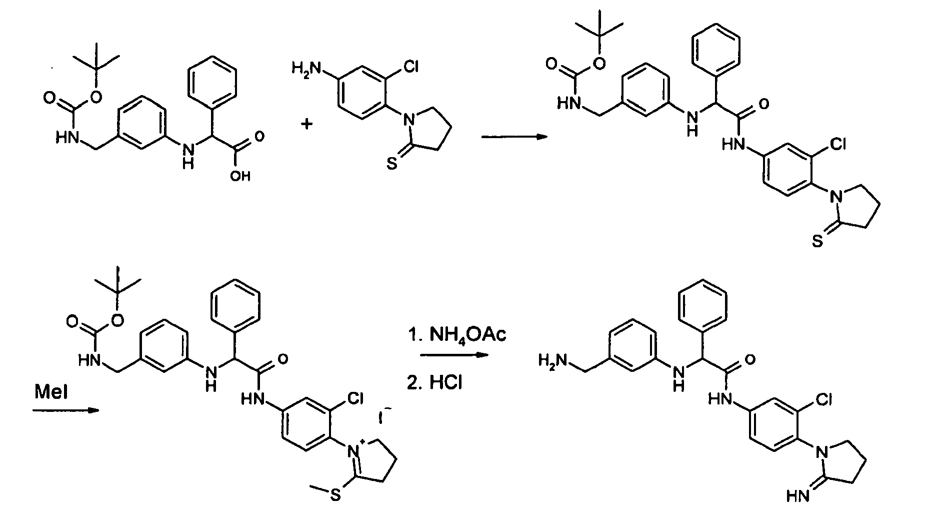

1.1 637 mg (2.81 mmol) 1-(4-Amino-2-methyl-phenyl)-piperidin-2-thion und 1g (2.81 mmol) (R,S)-[3-(*tert*-Butoxycarbonylamino-methyl)-phenyl-amino]-phenylessigsäure werden in 20 ml DMF gelöst und nacheinander mit 592.6 mg (3.09 mmol) N-(3-Dimethylaminopropyl)-N'-ethylcarbodiimid Hydrochlorid, 473.4 mg (2.81 mmol) 1-Hydroxybenzotriazol-Hydrat, sowie 1.24 ml (11.24 mmol) 4-Methylmorpholin versetzt. Nach 3 Tagen wird das Reaktionsgemisch in 100 ml Eiswasser eingerührt und vom Niederschlag abfiltriert. Nach Trocknung erhält man 1.17 g (2.07 mmol) (R,S)-[3-({[3-Chlor-4-(2-thioxo-pyrrolidin-1-yl)-phenylcarbamoyl]-phenyl-methyl}-amino)-benzyl]-carbaminsäure-*tert*-butyl-ester, ESI: 565, 567.
1.2 350 mg (0.62 mmol) (R,S)-[3-({[3-Chlor-4-(2-thioxo-pyrrolidin-1-yl)-phenylcarbamoyl]-phenyl-methyl}-amino)-benzyl]-carbaminsäure-tert-butylester werden in 10 ml wasserfreiem Aceton gelöst und mit 0.4 ml (0.68 mmol) lodmethan versetzt. Nach 48 Stunden wird das Reaktionsgemisch zur Trockene eingeengt. Man erhält 0.49 g (R,S)-1-(4-{2-[3-(tert-Butoxy-carbonylamino-methyl)-phenylamino]-2-phenyl-acetylamino}-2-chlorophenyl)-5-methylsulfanyl-3,4-dihydro-2*H*-pyrrolium Iodid als Rohprodukt, ESI: 579, 581.
1.3 490 mg (0.69 mmol) (R,S)-1-(4-{2-[3-(*tert*-Butoxycarbonylaminomethyl)-phenylamino]-2-phenyl-acetylamino}-2-chloro-phenyl)-5-methyl-sulfanyl-3,4-dihydro-2*H*-pyrrolium Iodid werden in 30 ml ketonfreiem Ethanol gelöst und mit 266 mg (3.45 mmol) Ammoniumacetat versetzt und zum Sieden erhitzt. Nach 1.5 Stunden wird abfiltriert und zur Trockene eingedampft. Nach Chromatographie erhält man 148 mg (0.27 mmol) [3-({[3-Chloro-4-(2-imino-pyrrolidin-1-yl)-phenylcarbamoyl]-phenyl-methyl}-amino)-benzyl]-carbaminsäure-*tert*-butyl ester. Dieses wird anschließend mit 4 ml HCl in Ether vesetzt. Nach 1.5 Stunden filtriert man ab und erhält 117 mg (0.24 mmol) (R,S)-2-(3-Aminomethyl-phenylamino)-*N*-[3-chloro-4-(2-imino-pyrrolidin-1-yl)-phenyl]-2-phenyl-acetamid, Hydrochlorid. ("AB"), ESI 448, 450.

Analog erhält man die nachstehenden Verbindungen
2-(3-Aminomethyl-phenylamino)-*N*-[3-methyl-4-(2-imino-piperidin-1-yl)-phenyl]-2-(2-fluorphenyl)-acetamid;
2-(3-Aminomethyl-phenylamino)-*N*-[3-chlor-4-(2-imino-pyrrolidin-1-yl)-phenyl]-2-(2-fluorphenyl)-acetamid;
2-(3-Aminomethyl-phenylamino)-*N*-[3-trifluormethyl-4-(2-azabicyclo[2.2.2]-octan-3-imino-2-yl)-phenyl]-2-(2-fluorphenyl)-acetamid;
2-(3-Aminomethyl-phenylamino)-*N*-[3-fluor-4-(2-imino-pyrrolidin-1-yl)-phenyl]-2-(2-chlorphenyl)-acetamid;
2-(3-Aminomethyl-phenylamino)-*N*-[3-methyl-4-(2-imino-pyrrolidin-1-yl)-phenyl]-2-(2-fluorphenyl)-acetamid;
2-(3-Aminomethyl-phenylamino)-*N*-[3-chlor-4-(2-imino-pyrrolidin-1-yl)-phenyl]-2-(2-chlorphenyl)-acetamid;
2-(3-Aminomethyl-phenylamino)-*N*-[3-methyl-4-(2-imino-pyrrolidin-1-yl)-phenyl]-2-phenyl-acetamid;
2-(3-Aminomethyl-phenylamino)-*N*-[3-methyl-4-(2-(2-dimethylamino-ethylimino)-pyrrolidin-1-yl)-phenyl]-2-phenyl-acetamid;
2-(3-Aminomethyl-phenylamino)-*N*-[3-methyl-4-(2-imino-pyrrolidin-1-yl)-phenyl]-2-(2-chlorphenyl)-acetamid;
2-(3-Aminomethyl-phenylamino)-*N*-[3-trifluormethyl-4-(2-azabicyclo[2.2.2]-octan-3-imino-2-yl)-phenyl]-2-(2-chlorphenyl)-acetamid.

### Beispiel 2

### Herstellung eines Aminbausteins:

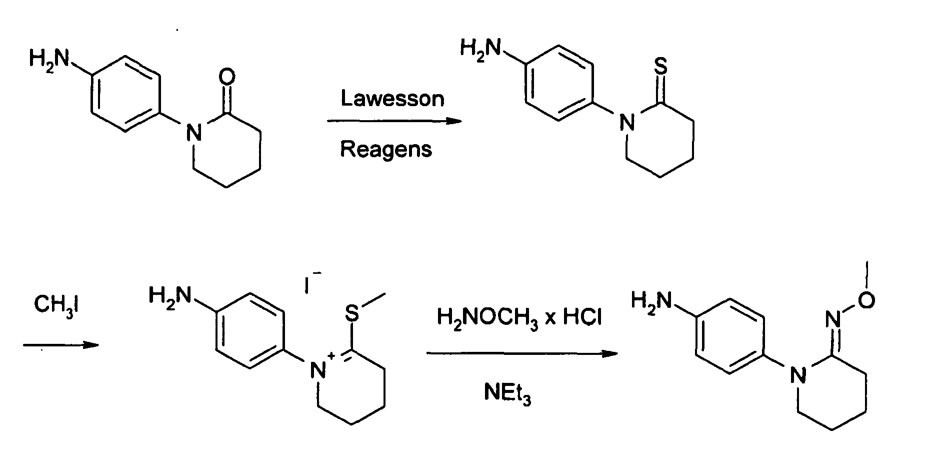

15 g (78.8 mmol) 1-(4-Aminophenyl)-piperidin-2-on werden zusammen mit 16.0 g (39.5 mmol) 2,4-Bis-(4-methoxy-phenyl)-[1,3,2,4]dithiadiphosphetan 2,4-disulfid (Lawesson-Reagens) in 100 ml wasserfreiem Toluol zum Sieden erhitzt. Nach 45 min. wird das Lösungsmittel verdampft und der Rückstand in Dichlormethan und 2 N HCl aufgenommen. Die wässrige
Phase wird dreimal mit Dichlormethan extrahiert und mit konz. NaOH auf einen pH-Wert von 12 eingestellt. Extraktion mit Dichlormethan, Trocknen über Natriumsulfat und Eindampfen des Lösungsmittels liefern 1-(4-Aminophenyl)-piperidin-2-thion als farblosen Feststoff, ESI 207.

Eine Lösung von 3.74 g (18.1 mmol) 1-(4-Amino-phenyl)-piperidin-2-thion in 30 ml Aceton wird mit 1.25 ml (20.0 mmol) lodmethan versetzt und 48 Stunden bei Raumtemperatur gerührt. Das Reaktionsgemisch wird eingedampft: 1-(4-Amino-phenyl)-6-methylsulfanyl-2,3,4,5-tetrahydro-pyridinium-iodid als bräunlicher Feststoff; ESI 221.

Eine Lösung von 2.68 g (12.1 mmol) 1-(4-Amino-phenyl)-6-methylsulfanyl-2,3,4,5-tetrahydropyridinium-iodid und 1.01 g (12.1 mmol) O-Methylhydroxylammoniumchlorid in 30 ml Ethanol wird mit 3.5 ml (25 mmol) Triethylamin versetzt und 20 Stunden bei Raumtemperatur gerührt. Das Reaktionsgemisch wird eingedampft, in Wasser aufgenommen und der entstandene Niederschlag abfiltriert: 1-(4-Amino-phenyl)-piperidin-2-on-O-methyl-oxim als farbloser Feststoff; ESI 220.

### Herstellung von 2-(1-Amino-isochinolin-7-yloxy)-N-(4-(2-imino-piperidin-1-yl)-phenyl]-4-methyl-valeriansäureamid:

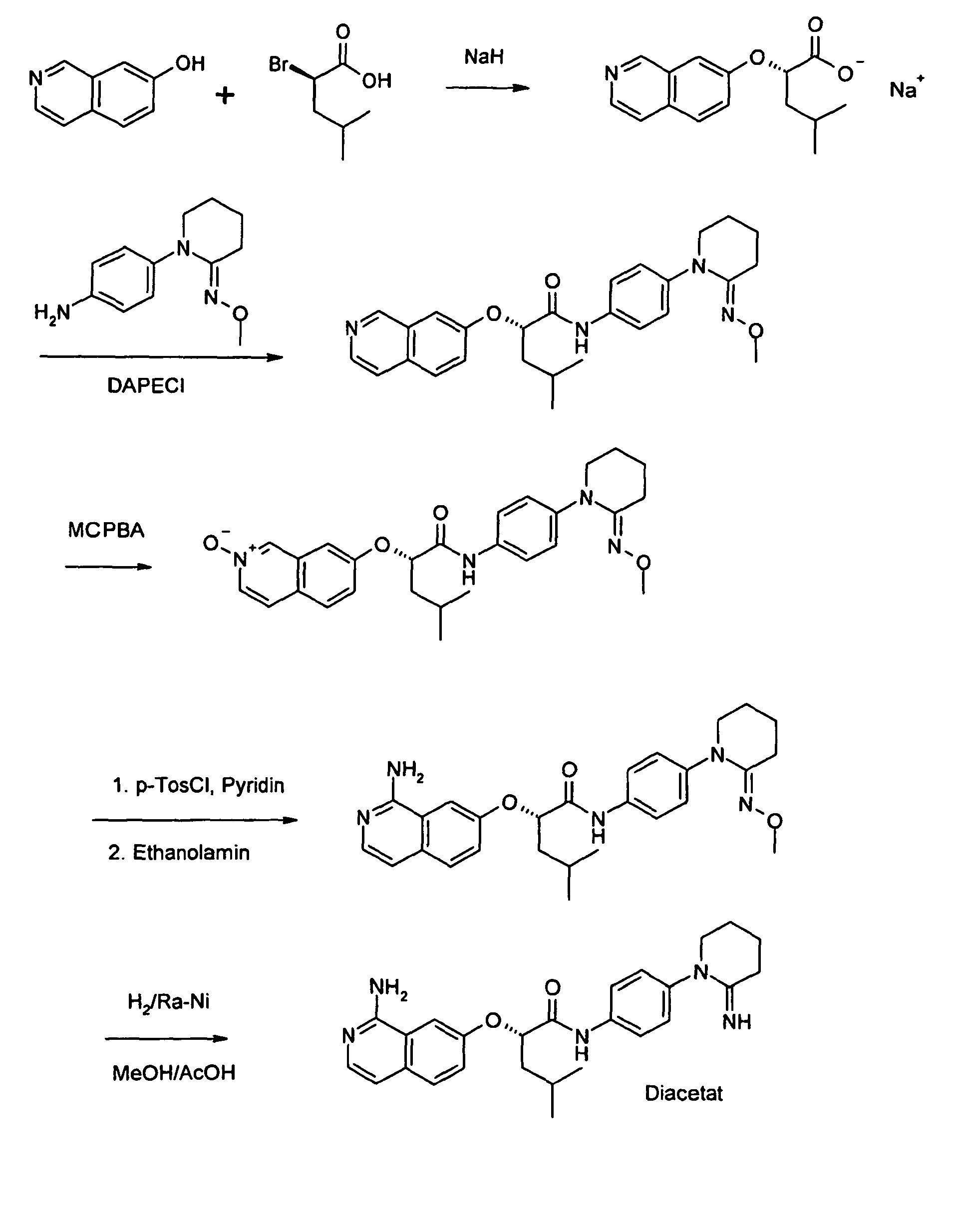

2.1 Eine Lösung von 5.00 g (34.4 mmol) 7-Hydroxyisochinolin und 6.72 g (34.4 mmol) (R)-2-Brom-4-methylpentansäure in 50 ml Tetrahydrofuran wird mit 2.76 g (68.9 mmol) Natriumhydrid, 60%ig in Paraffinöl, versetzt und 2 Stunden bei Raumtemperatur gerührt. Das Reaktionsgemisch wird eingedampft und mit heißem Acetonitril digeriert. Der Niederschlag wird abfiltriert: Rohes Natrium-(S)-2-(isochinolin-7-yloxy)-4-methyl-pentanoat (enthält noch Natriumbromid) als gelblicher Feststoff; ESI 260.
2.2 Eine Lösung von 384 mg (ca. 1.00 mmol) Natrium-(S)-2-(isochinolin-7-yloxy)-4-methyl-pentanoat, 219 mg (1.00 mmol) 1-(4-Aminophenyl)-piperidin-2-on-O-methyl-oxim, 249 mg (1.3 mmol) N-(3-Dimethylaminopropyl)-N'-ethylcarbodiimidhydrochlorid (DAPECI) und 135 mg (1.00 mmol) 1-Hydroxybenztriazol (HOBt) in 2 ml DMF wird 18 Stunden bei Raumtemperatur gerührt. Das Reaktionsgemisch wird auf gesättigte Natriumhydrogencarbonat-Lösung gegeben und mit Ethylacetat extrahiert. Die organische Phase wird über Natriumsulfat getrocknet und eingedampft: (S)-2-(Isochinolin-7-yloxy)-4-methyl-pentansäure-[4-(2-methoxyimino-piperidin-1-yl)-phenyl]-amid als farbloser Feststoff; ESI 461.
2.3 Eine Lösung von 360 mg (0.782 mmol) (S)-2-(lsochinolin-7-yloxy)-4-methyl-pentansäure-[4-(2-methoxyimino-piperidin-1-yl)-phenyl]-amid in 10 ml Dichlormethan wird mit 173 mg (1.00 mmol) 3-Chlorperbenzoesäure versetzt und 48 Stunden bei Raumtemperatur gerührt Das Reaktionsgemisch wird zwischen Dichlormethan und gesättigter Natriumhydrogencarbonatlösung verteilt. Die organische Phase wird eingedampft: (S)-4-Methyl-2-(2-oxy-isochinolin-7-yloxy)-pentansäure-[4-(2-methoxyimino-piperidin-1-yl)-phenyl]-amid als farbloser Feststoff; ESI 477
2.4 Eine Lösung von 370 mg (0.777 mmol) 4-Methyl-2-(2-oxy-isochinolin-7-yloxy)-pentansäure-[4-(2-methoxyimino-piperidin-1-yl)-phenyl]-amid in 1 ml Pyridin wird mit 191 mg (1.00 mmol) 4-Toluolsulfonylchlorid versetzt und 24 Stunden bei Raumtemperatur gerührt. Das Lösungsmittel wird abdestilliert, der Rückstand in 2 ml Ethanolamin gelöst und 42 Stunden bei Raumtemperatur gerührt. Das Reaktionsgemisch wird auf Wasser gegeben und mit Ethylacetat extrahiert. Die organische Phase wird eingedampft und der Rückstand an einer Kieselgelsäule chromatographiert: (S)-2-(1-Amino-isochinolin-7-yloxy)-N-[4-(2-imino-piperidin-1-yl)-phenyl]-4-methyl-valeriansäureamid ("BA") als farbloser Feststoff; ESI 476.
2.5 Eine Lösung von 50 mg (0.105 mmol) (S)-2-(1-Amino-isochinolin-7-yloxy)-4-methyl-pentansäure-[4-(2-methoxyimino-piperidin-1-yl)-phenyl]-amid in 10 ml Methanol wird mit 300 mg Raney-Nickel und 5 mg Essigsäure versetzt und bei Raumtemperatur und Normaldruck hydriert. Der Katalysator wird abfiltriert und das Filtrat eingedampft: (S)-2-(1-Amino-isochinolin-7-yloxy)-N-[4-(2-imino-piperidin-1-yl)-phenyl]-4-methyl-valerian-säureamid, Diacetat ("BB") als farbloser Feststoff; ESI 446.

Analog erhält man die nachstehenden Verbindungen
2-(1-Amino-isochinolin-7-yloxy)-*N*-[3-methyl-4-(2-imino-piperidin-1-yl)-phenyl]-4-methyl-valeriansäureamid;
2-(1-Amino-isochinolin-7-yloxy)-*N*-[3-methyl-4-(2-methoxyimino-piperidin-1-yl)-phenyl]-4-methyl-valeriansäureamid;
2-(3-Amino-benzo[d]-isoxazol-5-ylamino)-*N*-[3-chlor-4-(2-imino-pyrrolidin-1-yl)-phenyl]-2-phenyl-acetamid;
2-(1-Amino-isochinolin-7-yloxy)-*N*-[4-(2-imino-pyrrolidin-1-yl)-phenyl]-4-methyl-valeriansäureamid;
2-(1-Amino-isochinolin-7-yloxy)-*N*-[4-(2-methoxyimino-pyrrolidin-1-yl)-phenyl]-4-methyl-valeriansäureamid.
2-(3-Amino-1 *H*-indazol-5-ylamino)-*N*-[3-chlor-4-(2-imino-pyrrolidin-1-yl)-phenyl]-2-phenyl-acetamid.

### Beispiel 3

### Herstellung von 2-(3-Aminomethyl-phenylamino)-N-[3-chlor-4-(2-hydroxyimino-pyrrolidin-1-yl)-phenyl] 2-phenyl-acetamid

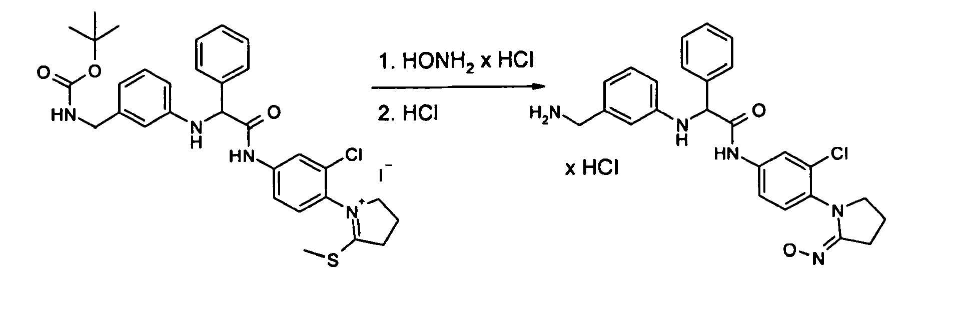

150 mg (0.21 mmol) (R,S)-1-(4-{2-[3-(*tert*-Butoxycarbonylamino-methyl)-phenylamino]-2-phenyl-acetylamino}-2-chloro-phenyl)-5-methylsulfanyl-3,4-dihydro-2H-pyrrolium Iodid werden in 10 ml ketonfreiem Ethanol gelöst und mit 14.59 mg (0.21 mmol) Hydroxylammonium Hydrochlorid sowie 0.06 ml (0.42 mmol) Triethylamin versetzt. Nach 20 Stunden wird zur Trockene eingedampft, der Rückstand in Wasser eingerührt und abfiltriert. Das Rohprodukt wird nach der Trocknung mit 20 ml HCl in Ether vesetzt. Nach 20 Stunden wird das Lösungsmittel im Vakuum entfernt und mit Ether ausgerührt. Man erhält 34 mg (0.07 mmol) (R,S)-2-(3-Aminomethyl-phenylamino)-*N*-[3-chloro-4-(2-hydroxyimino-pyrrolidin-1-yl)-phenyl]-2-phenyl-acetamid ("AA"), ESI 464.

Analog erhält man die nachstehenden Verbindungen
2-(3-Aminomethyl-phenylamino)-*N*-[3-methyl-4-(2-hydroxyimino-piperidin-1-yl)-phenyl]-2-(2-fluorphenyl)-acetamid;
2-(3-Aminomethyl-phenylamino)-*N*-[3-chlor-4-(2-hydroxyimino-pyrrolidin-1-yl)-phenyl]-2-(2-fluorphenyl)-acetamid;
2-(3-Aminomethyl-phenylamino)-*N*-[3-trifluormethyl-4-(2-azabicyclo[2.2.2]-octan-3-hydroxyimino-2-yl)-phenyl]-2-(2-fluorphenyl)-acetamid;

### Beispiel 4

### Herstellung von 2-(3-Aminomethyl-phenylamino)-N-[3-chlor-4-(2-thioxo-pyrrolidin-1-yl)-phenyl]-2-phenyl-acetamid

Analog Beispiel 1 erhält man durch Umsetzung der Thioxoaminkomponente mit der BOC-geschützten Carboxykomponente und anschließender Schutzgruppenabspaltung
2-(3-Aminomethyl-phenylamino)-N-[3-chlor-4-(2-thioxo-pyrrolidin-1-yl)-phenyl]-2-phenyl-acetamid;
und analog
2-(3-Aminomethyl-phenylamino)-N-[3-methyl-4-(2-thioxo-pyrrolidin-1-yl)-phenyl]-2-(2-chlorphenyl)-acetamid;
2-(3-Aminomethyl-phenylamino)-N-[3-fluor-4-(2-thioxo-pyrrolidin-1-yl)-phenyl]-2-(2-chlorphenyl)-acetamid;
2-(3-Aminomethyl-phenylamino)-N-[3-fluor-4-(2-thioxo-pyrrolidin-1-yl)-phenyl]-2-(2-fluorphenyl)-acetamid.

### Beispiel 5

### Herstellung von 2-(3-Aminomethyl-phenylamino)-N-[3-methyl-4-(2-(2-dimethylamino-ethylimino)-pyrrolidin-1-yl)-phenyl]-2-(2-chlor)-phenyl-acetamid ("DA"):

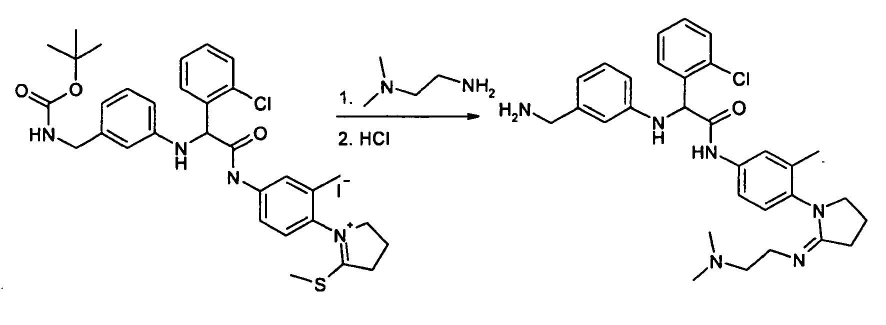

52 mg (0.07 mmol) (R,S)-1-(4-{2-[3-(*tert*-Butoxycarbonylamino-methyl)-phenylamino]-2-(2-chlor-phenyl)-acetylamino}-2-methyl-phenyl)-5-methylsulfanyl-3,4-dihydro-2*H*-pyrrolium Iodid werden in 10 ml ketonfreiem Ethanol gelöst und mit 0.04 ml (0.36 mmol) N,N-Dimethylethylendiamin versetzt und zum Sieden erhitzt. Nach 2 Stunden wird zur Trockene eingedampft, der Rückstand in 100 ml Ethylacetat aufgenommen und 2 mal mit je 30 ml gesättigter NaHCO₃-Lösung gewaschen. Nach Trocknen über NaSO₄ und Abdestillieren des Lösungsmittels erhält man 66 mg {3-[((2-Chlor-phenyl)-{4-[2-(2-dimethylamino-ethylimino)-pyrrolidin-1-yl]-3-methyl-phenylcarbamoyl}-methyl)-amino]-benzyl}-carbaminsäure-*tert*-butylester. Das Rohprodukt wird nach der Trocknung mit 10 ml HCl in Ether vesetzt. Nach 22 Stunden filtriert man ab und erhält 41 mg des Produkts "DA".

### Beispiel 6

### Die Herstellung von 2-(5-Amino-5,6,7,8-tetrahydro-naphthalin-2-yloxy)-N-[4-(3-imino-2-aza-bicyclo[2.2.2]oct-2-yl)-3-methyl-phenyl]-2-phenyl-acetamid erfolgt analog nachstehendem Schema:

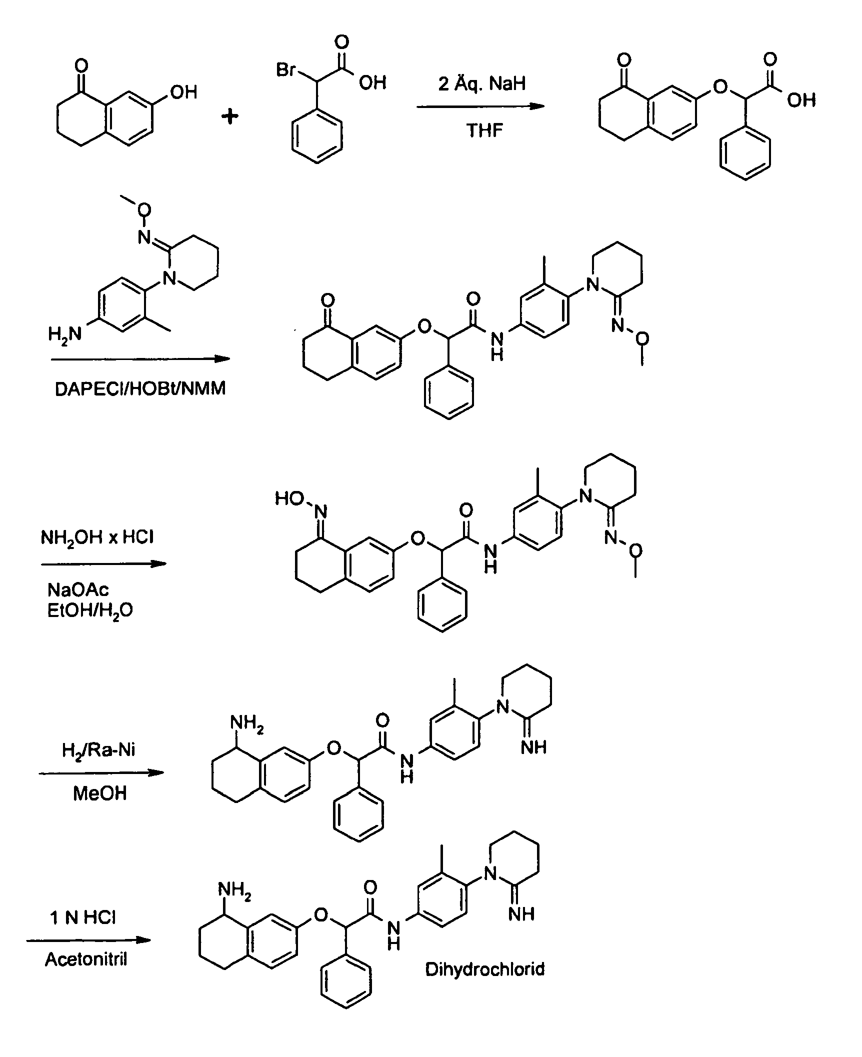

Analog erhält man die Verbindung
2-(5-Amino-5,6,7,8-tetrahydro-naphthalin-2-yloxy)-2-(2-fluor-phenyl)-N-[4-(3-imino-2-aza-bicyclo[2.2.2]oct-2-yl)-3-methyl-phenyl]-acetamid.

### Pharmakologische Daten

Affinität zu Rezeptoren

**Tabelle 1**

| Verbindung Nr. | FXa-IC₅₀ [M] | TF/FVIIa-IC₅₀ [M] |
|---|---|---|
| "AB" | 5.8 x 10⁻⁸ | 9.9 x 10⁻⁸ |
| "BA" | 6.8 × 10⁻⁷ | 4.9 x 10⁻⁷ |
| "BB" | 2.7 × 10⁻⁶ | 2.0 x 10⁻⁶ |
| "AA" | 2.2 × 10⁻⁷ | 2.9 x 10⁻⁷ |
| "DA" | 6.6 × 10⁻⁸ | 1.3 × 10⁻⁷ |
| | | |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ . 2 H₂O, 28,48 g Na₂HPO₄. 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 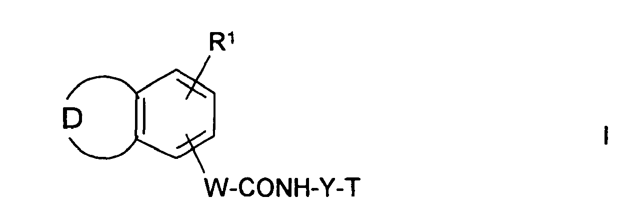
D fehlt oder
-CH=N-CH=CH-, -NH-N=CH-, -O-N=CH- oder
-CH₂-CH₂-CH₂-CH₂-,
und wobei, falls D vorhanden ist, zusätzlich an D eine einfache Substitution durch NH₂ auftreten kann,
R¹ H oder CH₂NH₂,
W -OC(R^{2a})₂- oder -NR²C(R^{2a})₂-,
R^{2a} H, A' oder Ar',
A' Alkyl mit 1, 2, 3, 4, 5 oder 6 C-Atomen und wobei 1-7 H-Atome durch F ersetzt sein können,
Ar' unsubstituiertes oder ein- oder zweifach durch Hal substituiertes Phenyl oder Benzyl,
Y unsubstituiertes oder ein- oder zweifach durch A, Cl oder F substituiertes Phenylen,
T einfach durch =NR^{2b}, =S oder =NOR^{2b} substituiertes Piperidin-1-yl, Pyrrolidin-1-yl, 1*H*-Pyridin-1-yl, Morpholin-4-yl, Piperazin-1-yl, 1,3-Oxazolidin-3-yl, 2*H*-Pyridazin-2-yl, Azepan-1-yl, 2-Aza-bicyclo[2.2.2]-octan-2-yl,
R^{2b} H, -CH₂CH₂NA'₂, OH oder OA",
A" Methyl, Ethyl, Propyl, Isopropyl oder Butyl
bedeuten,
sowie ihre pharmazeutisch verwendbaren Salze, Solvate und Stereoisomere, einschließlich deren Mischungen in allen Verhältnissen.

2. Verbindungen gemäß Anspruch 1 ausgewählt aus der Gruppe
2-(3-Aminomethyl-phenylamino)-*N*-[3-chlor-4-(2-hydroxyimino-pyrrolidin-1-yl)-phenyl]-2-phenyl-acetamid;
2-(3-Aminomethyl-phenylamino)-*N*-[3-chlor-4-(2-imino-pyrrolidin-1-yl)-phenyl]-2-phenyl-acetamid;
2-(1-Amino-isochinolin-7-yloxy)-*N*-[4-(2-methoxyimino-piperidin-1-yl)-phenyl]-4-methyl-valeriansäureamid;
2-(1-Amino-isochinolin-7-yloxy)-*N*-[4-(2-imino-piperidin-1-yl)-phenyl]-4-methyl-valeriansäureamid;
2-(3-Aminomethyl-phenylamino)-*N*-[3-methyl-4-(2-hydroxyimino-piperidin-1-yl)-phenyl]-2-(2-fluorphenyl)-acetamid;
2-(3-Aminomethyl-phenylamino)-*N*-[3-methyl-4-(2-imino-piperidin-1-yl)-phenyl]-2-(2-fluorphenyl)-acetamid;
2-(3-Aminomethyl-phenylamino)-N-[3-chlor-4-(2-hydroxyimino-pyrrolidin-1-yl)-phenyl]-2-(2-fluorphenyl)-acetamid;
2-(3-Aminomethyl-phenylamino)-*N*-[3-chlor-4-(2-imino-pyrrolidin-1-yl)-phenyl]-2-(2-fluorphenyl)-acetamid;
2-(1-Amino-isochinolin-7-yloxy)-*N*-[3-methyl-4-(2-imino-piperidin-1-yl)-phenyl]-4-methyl-valeriansäureamid;
2-(3-Aminomethyl-phenylamino)-N-[3-trifluormethyl-4-(2-aza-bicyclo[2.2.2]-octan-3-imino-2-yl)-phenyl]-2-(2-fluorphenyl)-acetamid;
2-(3-Aminomethyl-phenylamino)-*N*-[3-trifluormethyl-4-(2-aza-bicyclo[2.2 .2]-octan-3-hydroxyimino-2-yl)-phenyl]-2-(2-fluorphenyl)-acetamid;
2-(1-Amino-isochinolin-7-yloxy)-*N*-[3-methyl-4-(2-methoxy-imino-piperidin-1-yl)-phenyl]-4-methyl-valeriansäureamid;
2-(3-Aminomethyl-phenylamino)-*N*-[3-fluor-4-(2-imino-pyrrolidin-1-yl)-phenyl]-2-(2-chlorphenyl)-acetamid;
2-(3-Aminomethyl-phenylamino)-*N*-[3-methyl-4-(2-imino-pyrrolidin-1-yl)-phenyl]-2-(2-fluorphenyl)-acetamid;
2-(3-Aminomethyl-phenylamino)-*N*-[3-chlor-4-(2-imino-pyrrolidin-1-yl)-phenyl]-2-(2-chlorphenyl)-acetamid;
2-(3-Amino-benzo[d]-isoxazol-5-ylamino)-N-[3-chlor-4-(2-imino-pyrrolidin-1-yl)-phenyl]-2-phenyl-acetamid;
2-(1-Amino-isochinolin-7-yloxy)-*N*-[4-(2-imino-pyrrolidin-1-yl)-phenyl]-4-methyl-valeriansäureamid;
2-(1-Amino-isochinolin-7-yloxy)-*N*-[4-(2-methoxyimino-pyrrolidin-1-yl)-phenyl]-4-methyl-valeriansäureamid;
2-(3-Aminomethy)-phenylamino)-N-[3-methyl-4-(2-(2-dimethylamino-ethylimino)-pyrrolidin-1-yl)-phenyl]-2-(2-chlor)-phenyl-acetamid,
2-(5-Amino-5,6,7,8-tetrahydro-naphthalin-2-yloxy)-*N*-[4-(3-imino-2-aza-bicyclo[2.2.2]oct-2-yl)-3-methyl-phenyl]-2-phenyl-acetamid,
2-(5-Amino-5,6,7,8-tetrahydro-naphthalin-2-yloxy)-2-(2-fluorphenyl)-*N*-[4-(3-imino-2-aza-bicyclo[2.2.2]oct-2-yl)-3-methylphenyl]-acetamid,
sowie ihre pharmazeutisch verwendbaren Salze, Solvate und Stereoisomere, einschließlich deren Mischungen in allen Verhältnissen.

3. Verfahren zur Herstellung von Verbindungen der Formel I nach den Ansprüchen 1-2 sowie ihrer pharmazeutisch verwendbaren Salze, Solvate und Stereoisomere, **dadurch gekennzeichnet, daß** man
a) zur Herstellung einer Verbindung der Formel I, worin W -OC(R^{2a})₂- oder -NR^{2a}C(R^{2a})₂- bedeutet,
eine Verbindung der Formel II 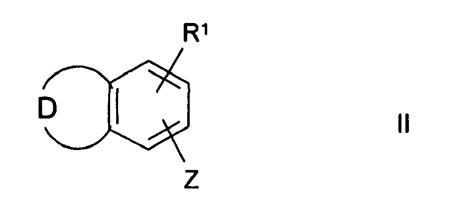 worin
Z OH oder NHR^{2a} bedeutet
und R¹, R^{2a} und D die in Anspruch 1 angegebenen Bedeutungen haben,
mit der Maßgabe, daß falls eine weitere OH- und/oder Aminogruppe vorliegt, diese geschützt ist,
mit einer Verbindung der Formel III
L-C(R^{2a})₂-CON H-Y-T III
worin
L Cl, Br oder I bedeutet und R^{2a}, Y und T die in Anspruch 1 angegebenen Bedeutungen haben,
umsetzt,
und anschließend gegebenenfalls eine Schutzgruppe abspaltet,
b) zur Herstellung einer Verbindung der Formel I,
eine Verbindung der Formel IV 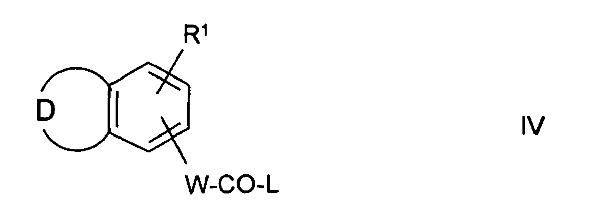 worin
L Cl, Br, I oder eine freie oder reaktionsfähig funktionell abgewandelte OH-Gruppe bedeutet bedeutet
und R¹, D und W die in Anspruch 1 angegebenen Bedeutungen haben,
mit der Maßgabe, daß falls eine weitere OH- und/oder Aminogruppe vorliegt, diese geschützt ist,
mit einer Verbindung der Formel V
Z'-Y-T V
worin
Z' NH₂ bedeutet
und Y und T die in Anspruch 1 angegebenen Bedeutungen haben, umsetzt,
und anschließend gegebenenfalls eine Schutzgruppe abspaltet,
c) und/oder daß man in einer Verbindung der Formel I einen Rest T und/oder R¹ in einen anderen Rest T und/oder R¹ umwandelt,
indem man beispielsweise
i) eine Sulfanylverbindung in eine Iminoverbindung umwandelt,
ii) eine Aminoschutzgruppe abspaltet,
und/oder
eine Base oder Säure der Formel I in eines ihrer Salze umwandelt.

4. Verbindungen der Formel I nach einem oder mehreren der Ansprüche 1 bis 2 als Inhibitoren des Koagulationsfaktors Xa.

5. Verbindungen der Formel I nach einem oder mehreren der Ansprüche 1 bis 2 als Inhibitoren des Koagulationsfaktors VIIa.

6. Arzneimittel, enthaltend mindestens eine Verbindung der Formel I nach einem oder mehreren der Ansprüche 1 bis 2 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

7. Arzneimittel enthaltend mindestens eine Verbindung der Formel I gemäß einem oder mehreren der Ansprüche 1 bis 2 und/oder ihre pharmazeutisch verwendbaren Salze, Solvate und Stereoisomere, einschließlich deren Mischungen in allen Verhältnissen, und mindestens einen weiteren Arzneimittelwirkstoff.

8. Verwendung von Verbindungen gemäß einem oder mehreren der Ansprüche 1 bis 2 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen.

9. Set (Kit), bestehend aus getrennten Packungen von
(a) einer wirksamen Menge an einer Verbindung der Formel I gemäß einem oder mehreren der Ansprüche 1 bis 2 und/oder ihrer pharmazeutisch verwendbaren Salze, Solvate und Stereoisomere, einschließlich deren Mischungen in allen Verhältnissen,
und
(b) einer wirksamen Menge eines weiteren Arzneimittelswirkstoffs.

10. Verwendung von Verbindungen der Formel gemäß einem oder mehreren der Ansprüche 1 bis 2 und/oder ihrer pharmazeutisch verwendbaren Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen,
in Kombination mit mindestens einem weiteren Arzneimittelwirkstoff.

11. Zwischenverbindungen der Formel VI 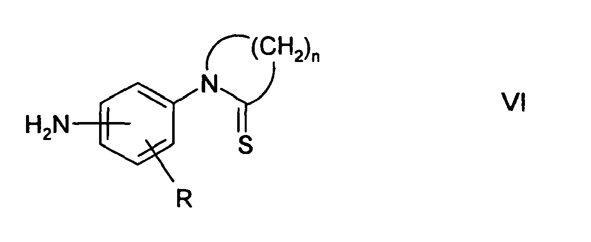 worin
R H, F, Cl oder A',
A' Alkyl mit 1-6 C-Atomen, wobei 1-7 H-Atome durch F ersetzt sein können,
n 3, 4 oder 5 bedeuten,
sowie deren Salze.

## Claims

1. Compounds of the formula I 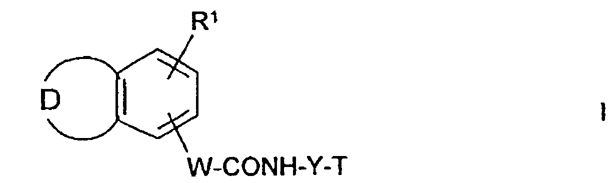 where
D is absent or
denotes -CH=N-CH=CH-, -NH-N=CH-, -O-N=CH- or
-CH₂-CH₂-CH₂-CH₂-,
and where, if D is present, a monosubstitution by NH₂ may additionally occur on D,
R¹ denotes H or CH₂NH₂,
W denotes -OC(R^{2a})₂- or -NR²C(R^{2a})₂-,
R^{2a} denotes H, A' or Ar',
A' denotes alkyl having 1, 2, 3, 4, 5 or 6 C atoms and where 1-7 H atoms may be replaced by F,
Ar' denotes phenyl or benzyl, each of which is unsubstituted or mono- or disubstituted by Hal,
Y denotes phenylene which is unsubstituted or mono- or disubstituted by A, Cl or F,
T denotes piperidin-1-yl, pyrrolidin-1-yl, 1*H*-pyridin-1-yl, morpholin-4-yl, piperazin-1-yl, 1,3-oxazolidin-3-yl, 2H-pyridazin-2-yl, azepan-1-yl, 2-azabicyclo[2.2.2]octan-2-yl, each of which is monosubstituted by =NR^{2b}, =S or =NOR^{2b},
R^{2b} denotes H, -CH₂CH₂NA'₂, OH or OA",
A" denotes methyl, ethyl, propyl, isopropyl or butyl,
and pharmaceutically usable salts, solvates and stereoisomers thereof, including mixtures thereof in all ratios.

2. Compounds according to Claim 1, selected from the group
2-(3-aminomethylphenylamino)-*N*-[3-chloro-4-(2-hydroxy-iminopyrrolidin-1-yl)phenyl]-2-phenylacetamide;
2-(3-aminomethylphenylamino)-*N*-(3-chloro-4-(2-iminopyrroli-din-1-yl)phenyl]-2-phenylacetamide;
2-(1-aminoisoquinolin-7-yloxy)-*N*-[4-(2-methoxyiminopiperi-din-1-yl)phenyl]-4-methylvaleramide;
2-(1-aminoisoquinolin-7-yloxy)-*N*-[4-(2-iminopiperidin-1 -yl)-phenyl]-4-methylvaleramide;
2-(3-aminomethylphenylamino)-*N*-[3-methyl-4-(2-hydroxy-iminopiperidin-1-yl)phenyl]-2-(2-fluorophenyl)acetamide;
2-(3-aminomethylphenylamino)-*N*-[3-methyl-4-(2-iminopiperi-din-1-yl)phenyl]-2-(2-fluorophenyl)acetamide;
2-(3-aminomethylphenylamino)-*N*-[3-chloro-4-(2-hydroxy-iminopyrrolidin-1-yl)phenyl]-2-(2-fluorophenyl)acetamide;
2-(3-aminomethylphenylamino)-*N*-[3-chloro-4-(2-iminopyrroli-din-1-yl)phenyl]-2-(2-fluorophenyl)acetamide;
2-(1-aminoisoquinolin-7-yloxy)-*N*-[3-methyl-4-(2-iminopiperi-din-1-yl)phenyl]-4-methylvaleramide;
2-(3-aminomethylphenylamino)-*N*-[3-trifluoromethyl-4-(2-aza-bicyclo[2.2.2]octan-3-imino-2-yl)phenyl]-2-(2-fluorophenyl)acetamide;
2-(3-aminomethylphenylamino)-N-[3-trifluoromethyl-4-(2-aza-bicyclo[2.2.2]octan-3-hydroxyimino-2-yl)phenyl]-2-(2-fluorophenyl)-acetamide;
2-( 1-aminoisoquinolin-7-yloxy)-N-[3-methyl-4-(2-methoxy-iminopiperidin-1-yl)phenyl]-4-methylvaleramide;
2-(3-aminomethylphenylamino)-N-[3-fluoro-4-(2-iminopyrroli-din-1-yl)phenyl]-2-(2-chlorophenyl)acetamide;
2-(3-aminomethylphenylamino)-*N*-[3-methyl-4-(2-iminopyrrolidin-1-yl)phenyl]-2-(2-fluorophenyl)acetamide;
2-(3-aminomethylphenylamino)-*N*-[3-chloro-4-(2-iminopyrrolidin-1-yl)phenyl]-2-(2-chlorophenyl)acetamide;
2-(3-aminobenzo[d]isoxazol-5-ylamino)-*N*-[3-chloro-4-(2-iminopyrrolidin-1-yl)phenyl]-2-phenylacetamide;
2-(1-aminoisoquinolin-7-yloxy)-*N*-[4-(2-iminopyrrolidin-1-yl)-phenyl]-4-methylvaleramide;
2-(1-aminoisoquinolin-7-yloxy)-*N*-[4-(2-methoxyiminopyrrolidin-1-yl)phenyl]-4-methylvaleramide;
2-(3-aminomethylphenylamino)-N-[3-methyl-4-(2-(2-dimethyl-aminoethylimino)pyrrolidin-1-yl)phenyl]-2-(2-chloro)phenylacet-amide;
2-(5-amino-5,6,7,8-tetrahydronaphthalen-2-yloxy)-*N*-[4-(3-imino-2-azabicyclo[2.2.2]oct-2-yl)-3-methylphenyl]-2-phenylacet-amide;
2-(5-amino-5,6,7,8-tetrahydronaphthalen-2-yloxy)-2-(2-fluorophenyl)-*N*-[4-(3-imino-2-azabicyclo[2.2.2]oct-2-yl)-3-methylphenyl]-acetamide;
and pharmaceutically usable salts, solvates and stereoisomers thereof, including mixtures thereof in all ratios.

3. Process for the preparation of compounds of the formula I according to Claims 1-2 and pharmaceutically usable salts, solvates and stereoisomers thereof, **characterised in that**
a) for the preparation of a compound of the formula I in which W denotes -OC(R^{2a})₂- or -NR^{2a}C(R^{2a})₂-,
a compound of the formula II 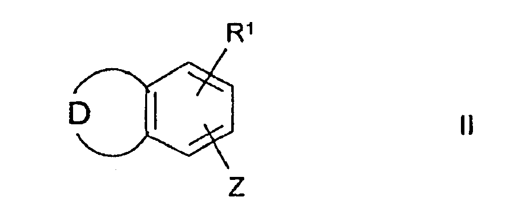 in which
Z denotes OH or NHR^{2a}
and R¹, R^{2a} and D have the meanings indicated in Claim 1,
with the proviso that any further OH and/or amino group present is protected,
is reacted with a compound of the formula III
L-C(R^{2a})₂-CONH-Y-T III
in which
L denotes Cl, Br or I and R^{2a}, Y and T have the meanings indicated in Claim 1,
and, where appropriate, a protecting group is subsequently removed,
b) for the preparation of a compound of the formula I,
a compound of the formula IV 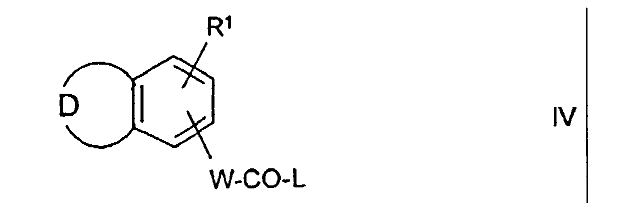 in which
L denotes Cl, Br, I or a free or reactively functionally modified OH group
and R¹, D and W have the meanings indicated in Claim 1,
with the proviso that any further OH and/or amino group present is protected,
is reacted with a compound of the formula V
Z'-Y-T V
in which
Z' denotes NH₂
and Y and T have the meanings indicated in Claim 1,
and, where appropriate, a protecting group is subsequently removed,
c) and/or **in that** a radical T and/or R¹ in a compound of the formula I is converted into another radical T and/or R¹
by, for example,
i) converting a sulfanyl compound into an imino compound,
ii) removing an amino-protecting group,
and/or
a base or acid of the formula I is converted into one of its salts.

4. Compounds of the formula I according to one or more of Claims 1 to 2 as inhibitors of coagulation factor Xa.

5. Compounds of the formula I according to one or more of Claims 1 to 2 as inhibitors of coagulation factor VIIa.

6. Medicaments comprising at least one compound of the formula I according to one or more of Claims 1 to 2 and/or pharmaceutically usable salts, solvates and stereoisomers thereof, including mixtures thereof in all ratios, and optionally excipients and/or adjuvants.

7. Medicaments comprising at least one compound of the formula I according to one or more of Claims 1 to 2 and/or pharmaceutically usable salts, solvates and stereoisomers thereof, including mixtures thereof in all ratios, and at least one further medicament active ingredient.

8. Use of compounds according to one or more of Claims 1 to 2 and/or physiologically acceptable salts and solvates thereof for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases.

9. Set (kit) consisting of separate packs of
(a) an effective amount of a compound of the formula I according to one or more of Claims 1 to 2 and/or pharmaceutically usable salts, solvates and stereoisomers thereof, including mixtures thereof in all ratios,
and
(b) an effective amount of a further medicament active ingredient.

10. Use of compounds of the formula I according to one or more of Claims 1 to 2 and/or pharmaceutically usable salts, solvates and stereoisomers thereof, including mixtures thereof in all ratios, for the preparation of a medicament for the treatment of thromboses, myocardial infarction, arteriosclerosis, inflammation, apoplexy, angina pectoris, restenosis after angioplasty, claudicatio intermittens, migraine, tumours, tumour diseases and/or tumour metastases, in combination with at least one further medicament active ingredient.

11. Intermediate compounds of the formula VI 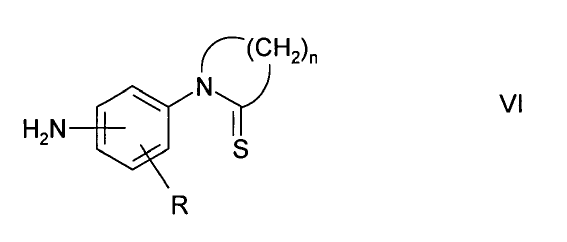 in which
R denotes H, F, Cl or A',
A' denotes alkyl having 1-6 C atoms, where 1-7 H atoms may be replaced by F,
n denotes 3, 4 or 5,
and salts thereof.

## Revendications

1. Composés de la formule I 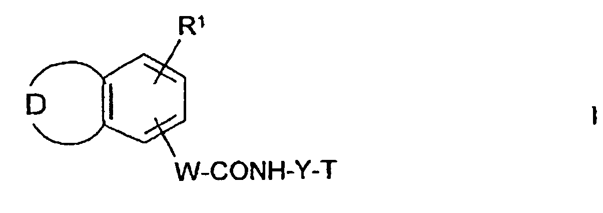 dans laquelle
D est absent ou
représente -CH=N-CH=CH-, -NH-N=CH-, -O-N=CH- ou
-CH₂-CH₂-CH₂-CH₂-,
et dans laquelle, si D est présent, une monosubstitution par NH₂ peut de façon additionnelle se produire sur D,
R¹ représente H ou CH₂NH₂,
W représente -OC(R^{2a})₂- ou -NR²C(R^{2a})₂-,
R^{2a} représente H, A' ou Ar',
A' représente alkyle comportant 1, 2, 3, 4, 5 ou 6 atomes de C et où 1-7 atomes de H peuvent être remplacés par F,
Ar' représente phényle ou benzyle, dont chacun est non substitué ou mono- ou disubstitué par Hal,
Y représente phénylène qui est non substitué ou mono- ou disubstitué par A, Cl ou F,
T représente pipéridine-1-yl, pyrrolidine-1-yl, 1*H*-pyridine-1-yl, morpholine-4-yl, pipérazine-1-yl, 1,3-oxazolidine-3-yl, 2H-pyridazine-2-yl, azepan-1-yl, 2-azabicyclo[2.2.2]octan-2-yl dont chacun est monosubstitué par =NR^{2b}, =S ou =NOR^{2b},
R^{2b} représente H, -CH₂CH₂NA'₂, OH ou OA",
A" représente méthyle, éthyle, propyle, isopropyle ou butyle,
et leurs sels, leurs solvates et leurs stéréoisomères pharmaceutiquement utilisables, y compris des mélanges afférents selon tous les rapports.

2. Composés selon la revendication 1, choisis parmi le groupe
2-(3-aminométhylphénylamino)-N-[3-chloro-4-(2-hydroxy-iminopyrrolidine-1-yl)phényl]-2-phénylacétamide;
2-(3-aminométhylphénylamino)-N-[3-chloro-4-(2-iminopyrrolidine-1-yl)phényl]-2-phénylacétamide;
2-(1-aminoisoquinoline-7-yloxy)-*N*-[4-(2-méthoxyiminopipéridine-1-yl)phényl]-4-méthylvaléramide;
2-(1-aminoisoquinoline-7-yloxy)-*N*-[4-(2-iminopipéridine-1-yl)phényl]-4-méthylvaléramide;
2-(3-aminométhylphénylamino)-*N*-[3-méthyl-4-(2-hydroxy-iminopipéridine-1-yl)phényl]-2-(2-fluorophényl)acétamide;
2-(3-aminométhylphénylamino)-*N*-[3-méthyl-4-(2-iminopipéridine-1-yl)phényl]-2-(2-fluorophényl)acétamide;
2-(3-aminométhylphénylamino)-*N*-[3-chloro-4-(2-hydroxy-iminopyrrolidine-1-yl)phényl]-2-(2-fluorophényl)acétamide;
2-(3-aminométhylphénylamino)-*N*-[3-chloro-4-(2-iminopyrrolidine-1-yl)phényl]-2-(2-fluorophényl)acétamide;
2-(1-aminoisoquinolin-7-yloxy)-*N*-[3-méthyl-4-(2-iminopipéridine-1-yl)phényl]-4-méthylvaléramide;
2-(3-aminométhylphénylamino)-*N*-[3-trifluorométhyl-4-(2-azabicyclo[2.2.2]octan-3-imino-2-yl)phényl]-2-(2-fluorophényl)-acétamide;
2-(3-aminométhylphénylamino)-*N*-[3-trifluorométhyl-4-(2-azabicyclo[2.2.2]octan-3-hydroxyimino-2-yl)phényl]-2-(2-fluoro-phényl)acétamide;
2-(1-aminoisoquinolin-7-yloxy)-*N*-[3-méthyl-4-(2-méthoxy-iminopipéridine-1-yl)phényl]-4-méthylvaléramide;
2-(3-aminométhylphénylamino)-*N*-[3-fluoro-4-(2-iminopyrroli-dine-1-yl)phényl]-2-(2-chlorophényl)acétamide;
2-(3-aminométhylphénylamino)-*N*-[3-méthyl-4-(2-iminopyrrolidine-1-yl)phényl]-2-(2-fluorophényl)acétamide;
2-(3-aminométhylphénylamino)-N-[3-chloro-4-(2-iminopyrrolidine-1-yl)phényl]-2-(2-chlorophényl)acétamide;
2-(3-aminobenzo[d]isoxazol-5-ylamino)-*N*-[3-chloro-4-(2-iminopyrrolidine-1-yl)phényl]-2-phénylacétamide;
2-(1-aminoisoquinoline-7-yloxy)-*N*-[4-(2-iminopyrrolidine-1-yl)phényl]-4-méthylvaléramide;
2-(1-aminoisoquinoline-7-yloxy)-*N*-[4-(2-méthoxyiminopyrrolidine-1-yl)phényl]-4-méthylvaléramide;
2-(3-aminométhylphénylamino)-N-[3-méthyl-4-(2-(2-diméthyl-aminoéthylimino)pyrrolidine-1-yl)phényl]-2-(2-chloro)phénylacétamide;
2-(5-amino-5,6,7,8-tétrahydronaphthalène-2-yloxy)-*N*-[4-(3-imino-2-azabicyclo[2.2.2]oct-2-yl)-3-méthylphényl]-2-phénylacétamide;
2-(5-amino-5,6,7,8-tétrahydronaphthalène-2-yloxy)-2-(2-fluorophényle)-N-[4-(3-imino-2-azabicyclo[2.2.2]oct-2-yl)-3-méthyl-phényl]acétamide;
et leurs sels, leurs solvates et leurs stéréoisomères pharmaceutiquement utilisables, y compris des mélanges afférents selon tous les rapports.

3. Procédé pour la préparation de composés de la formule I selon les revendications 1-2 et de leurs sels, leurs solvates et leurs stéréoisomères pharmaceutiquement utilisables, **caractérisé en ce que**
a) pour la préparation d'un composé de la formule I dans laquelle W représente -OC(R^{2a})₂- ou -NR^{2a}C(R^{2a})₂-,
un composé de la formule II 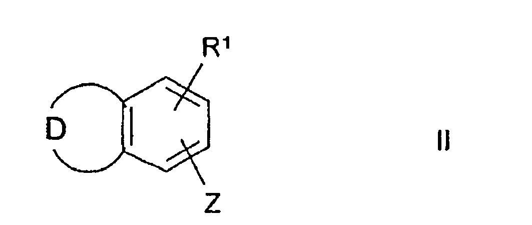 dans laquelle
Z représente OH ou NHR^{2a}
et R¹, R^{2a} et D présentent les significations indiquées selon la revendication 1,
étant entendu qu'un quelconque OH supplémentaire et/ou qu'un quelconque groupe amino supplémentaire présent est protégé,
est amené à réagir avec un composé de la formule III
L-C (R^{2a})₂-CONH-Y-T III
dans laquelle
L représente CI, Br ou I et R^{2a}, Y et T présentent les significations indiquées selon la revendication 1,
et, lorsque approprié, un groupe de protection est ensuite enlevé,
b) pour la préparation d'un composé de la formule I,
un composé de la formule IV 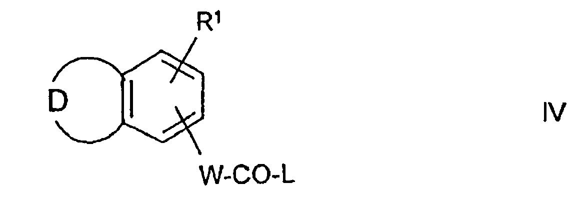 dans laquelle
L représente Cl, Br, I ou un groupe OH libre ou réactivement fonctionnellement modifié
et R¹, D et W présentent les significations indiquées selon la revendication 1,
étant entendu qu'un quelconque OH supplémentaire et/ou qu'un quelconque groupe amino supplémentaire présent est protégé,
est amené à réagir avec un composé de la formule V
Z'-Y-T V
dans laquelle
Z' représente NH₂
et Y et T présentent les significations indiquées selon la revendication 1,
et, lorsque approprié, un groupe de protection est ensuite enlevé,
c) et/ou **en ce qu'**un radical T et/ou R¹ selon un composé de la formule I est converti selon un autre radical T et/ou R¹
en, par exemple,
i) convertissant un groupe sulfanyle selon un composé imino,
ii) enlevant un groupe de protection amino,
et/ou
une base ou un acide de la formule I est converti selon l'un de ses sels.

4. Composés de la formule I selon une ou plusieurs des revendications 1 à 2 en tant qu'inhibiteurs d'un facteur de coagulation Xa.

5. Composés de la formule I selon une ou plusieurs des revendications 1 à 2 en tant qu'inhibiteurs d'un facteur de coagulation VIIa.

6. Médicaments comprenant au moins un composé de la formule I selon une ou plusieurs des revendications 1 à 2 et/ou leurs sels, leurs solvates et leurs stéréoisomères pharmaceutiquement utilisables, y compris des mélanges afférents selon tous les rapports, et en option, des excipients et/ou des adjuvants.

7. Médicaments comprenant au moins un composé de la formule I selon une ou plusieurs des revendications 1 à 2 et/ou leurs sels, leurs solvates et leurs stéréoisomères pharmaceutiquement utilisables, y compris des mélanges afférents selon tous les rapports, et au moins un autre ingrédient actif de médicaments.

8. Utilisation de composés selon une ou plusieurs des revendications 1 à 2 et/ou de leurs sels et de leurs solvates acceptables physiologiquement pour la préparation d'un médicament pour le traitement des thromboses, de l'infarctus du myocarde, de l'artériosclérose, de l'inflammation, de l'apoplexie, de l'angine de poitrine, de la resténose après angioplastie, de la claudication intermittente, de la migraine, des tumeurs, des maladies tumorales et/ou des métastases tumorales.

9. Jeu (kit) constitué par des groupements séparés de
(a) une quantité efficace d'un composé de la formule I selon une ou plusieurs des revendications 1 à 2 et/ou de leurs sels, de leurs solvates et de leurs stéréoisomères pharmaceutiquement utilisables, y compris des mélanges afférents selon tous les rapports,
et
(b) une quantité efficace d'un autre ingrédient actif de médicament.

10. Utilisation de composés de la formule 1 selon une ou plusieurs des revendications 1 à 2 et/ou de leurs sels, de leurs solvates et de leurs stéréoisomères pharmaceutiquement utilisables, y compris des mélanges afférents selon tous les rapports, pour la préparation d'un médicament pour le traitement des thromboses, de l'infarctus du myocarde, de l'artériosclérose, de l'inflammation, de l'apoplexie, de l'angine de poitrine, de la resténose après angioplastie, de la claudication intermittente, de la migraine, des tumeurs, des maladies tumorales et/ou des métastases tumorales,
en combinaison avec au moins un autre ingrédient actif de médicament.

11. Composés intermédiaires de la formule VI 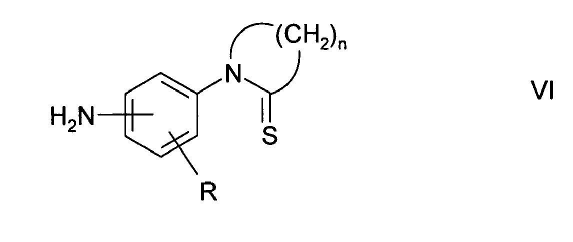 dans laquelle
R représente H, F, Cl ou A',
A' représente alkyle comportant de 1 à 6 atomes de C, où de 1 à 7 atomes de H peuvent être remplacés par F,
n représente 3, 4 ou 5,
et leurs sels.
